# EUROPEAN PATENT APPLICATION

(11) **EP 2 031 527 A1**
(43) Date of publication of application: **04.03.2009**
(21) Application number: 07405222.6
(22) Date of filing: 27.07.2007
(51) Int. Cl.: G06F 19/00

(54) **Portable medical device**

(71) Applicant: Sensile Pat AG, 6340 Baar (CH)
(72) Inventor: Brandt, Derek, 4436 Oberdorf (CH); Calasso, Irio, 6415 Arth (CH); Lüdi, Christoph, 3048 Worblaufen (CH)
(74) Representative: Reuteler, Raymond Werner

(57) **Abstract**

A medical device (1) comprising a memory (21), a processor (20), a medical device interface (2), and a program configured to process data obtained or generated by the medical device (1) into a format readable by a personal computer browser or spreadsheet program. The program further is configured to automatically initiate the computer browser or spreadsheet program of a personal computer (5) to display the processed data using standard software when the medical device interface (2) is connected to a personal computer interface (4).

## Description

The present invention relates to a portable medical device, particularly to a portable drug delivery device or a portable analyte sensor device.

For patients using medical devices, such as drug delivery devices or analyte sensor devices, it is crucial to monitor the doses delivered or the values measured in order to survey or adjust the medical treatment. For instance, with diabetes patients the delivery of insulin should follow a delivery program which depends on the time of day, the nutrition intake, type of diabetes, and personal characteristics of the patient. Not only is the correct dosing and timing of insulin delivery of importance, but also the constant monitoring of the doses already delivered. This is of particular importance when the delivery program has to be adjusted or changed. With regard to the above, the management of data obtained by the medical device, often referred to as "electronic diaries", is important for the treatment of patients using medical devices. Such electronic diaries may consist of values and points in time of medical parameters such as blood glucose concentration, cholesterol level, blood clotting parameter, blood pressure, or parameters for the injection of medicine, such as insulin pumps or insulin pens.

Desirably, the monitoring of medical parameters is performed not only in hospitals or medical centres, but also in daily life. Accordingly patients often need to manage their electronic diaries themselves, and in places other than hospitals or medical centres.

A solution to manage electronic diaries of medical devices has been proposed, where the medical device communicates wirelessly with a computer in order to transfer the data obtained by the medical device to the computer, and where software installed on the computer then processes the data. This solution however requires a computer with the necessary specialized software. For patients using the medical device at home, software installation is time-consuming and is often felt to be troublesome. Furthermore, patients who wish to manage their medical device with any other computer than with their own computer, have to bring the corresponding specialized software and install it on every computer they wish to manage their medical device. This is inconvenient, and at the same time sensitive data might be saved on a foreign computer without the consent of the patient. Moreover, administrator rights often are required to install new software, so that in many cases, a patient may be prevented from managing his medical device on a foreign computer.

Another solution that has been developed for the patient to manage his electronic diary is to directly use a monitor contained on the surface of the medical device. However, as the medical device should be as small as possible for more convenient use, the displays found on such medical devices are normally small and inconvenient. Such medical devices containing user interfaces (buttons and monitor) are either not convenient to carry because of their size, or they are not capable of displaying complex graphs. With the few buttons typically provided on such medical devices, it is inconvenient and sometimes impossible to manage the electronic diary, e.g. to write comments to occurring incidents or to determine a complex program of drug administration. Furthermore, medical devices attached to the user's skin, e.g. insulin patch pumps, are difficult to access when being in use.

The document EP 1758039 describes a communication adapter, which communicates with a medical device using IR on one side and which is connected to a computer using an interface and a cable connection on the other side. The software for the processing of the data obtained by the medical device is contained in the communication adapter, so that no specialized software on the computer is necessary. Several different medical devices can communicate with the communication adapter.

This solution allows the patient to manage his electronic diary, however it has several drawbacks. Firstly, the patient will not always carry the communication adapter, so that the entireness of the collected data and the software for processing the data is not automatically with the patient and his medical device. The communication adapter is more likely to stay attached to the patient's home computer, so that a foreign computer cannot be used to manage the medical device. Secondly, as there are two separate devices, the patient has to remember carrying both devices, which is inconvenient, especially when travelling. Thirdly, the IR connection between the communication unit and the medical device requires that the two devices are correctly positioned in respect to each other, so that the medical device normally has to be dismounted from the patient. Furthermore, alien signals could derange the IR connection between the medical device and the communication unit, i.e. the transfer of data between the medical device and the computer is more error-prone than necessary. Moreover, additional power for the radiation of the IR signal is consumed, which is especially relevant for the medical device and its limited power resources.

In view of the above, an object of the invention is to provide a portable medical device which overcomes the above-mentioned drawbacks of the present medical device management solutions.

It is an object of the invention to provide a portable medical device that can be managed with a user interface in an easy, safe, and convenient way.

It would be advantageous to provide a portable medical device that is versatile to use, has a high autonomy, and is convenient to carry.

Objects of the invention are achieved by a medical device according to claim 1.

There is now provided a portable medical device comprising a memory, a processor, a plug and play medical device interface, and a program configured to process data obtained or generated by the medical device into a format readable by a personal computer browser or spreadsheet program.

The program is preferably configured to automatically initiate the computer browser or spreadsheet program of a personal computer to display the processed data using standard software when the medical device interface is connected to a computer interface.

The plug and play interface advantageously enables automatic configuration of the medical device on connection to a personal computer interface, such that the medical device is automatically recognised by the personal computer, and configured to enable data exchange therewith, without requiring any reconfiguration or installation of device drivers.

The portable medical device contains a program that initiates the computer to run a non-specialized program upon connecting the medical device directly to a personal computer, such that the personal computer serves as a user interface for managing the medical device. As the program contained in the medical device is able to process the data obtained by the medical device in such a way that the data can be transferred to the personal computer and displayed with a non-specialized program normally present on personal computers, such as internet browsers or spreadsheet programs, there is no need to install additional programs on the personal computer. Typical examples of internet browsers commonly installed on personal computers are the browsers known under the tradenames "Firefox", "Internet Explorer", "Netscape", or "Mozilla". A typical example of a spreadsheet program is the spreadsheet program known under the tradename "Microsoft Office Excel". A non-specialized program for the purpose of the present invention is a program that would be installed on a regular personal computer, that is not dedicated to, or is not intended to be used for, managing a medical device. A personal computer in the sense of this invention may be any personal computer, a cellular phone, palm-top, PDA, other electronic organizer or the like that are intended to be used for applications such as word processing, electronic mail exchange, spreadsheet processing, electronic diary processing, not specifically dedicated to medical applications.

Another embodiment of the present invention provides a portable medical device which contains a program capable of displaying data stored on the medical device on a personal computer i.e. instead of using a specialized program installed on a personal computer dedicated for the medical device. In this case, the program stored on the medical device is run from the personal computer when the medical device is connected to the personal computer. In order to ensure compatibility with different operating systems found on personal computers, multiple such displaying programs are provided on the medical device. Accordingly the medical device preferably includes a display program compatible with each known operating system, e.g. for the programs known under the tradenames "Microsoft Windows", "Mac OS", "Linux " and other commonly used operating system programs.

Such systems are also referred to as "real plug and play" systems, where the user only has to establish the connection in order to start a program. Compared to solutions where additional software needs to be installed, the present invention ensures ease of use and convenience for the patient.

Spreadsheet programs and internet browsers on a personal computer are capable of displaying more complex figures and graphs than what is possible to display on a small display contained on a medical device. Keyboard and mouse further facilitate the management of the medical device.

The above described features ensure convenience when managing the medical device. The inhibition threshold present in some patients to use a computer for managing their medical device may be considerably lowered.

The portable medical device according to the present invention allows for additional functions benefiting the user. For instance, the safety of the medical device may be significantly improved, as information on the patient's condition or on the medical treatment the patient receives may be sent to a doctor or a medical centre when the medical device is connected to a personal computer which is connected to a web such as the internet. According to one embodiment of the present invention, an alarm function is provided wherein the patient receives a warning signal when the medical device detects a value that lies outside a predefined range. Said range may be adjusted when the medical device is connected to the personal computer, e.g. by the doctor or medical centre. Furthermore the patient carries information relevant for his health condition with him; as such information is stored on the medical device. Accordingly, e.g. in the case of an accident, a person arriving at the accident may simply connect the medical device to any personal computer and have insight in information stored on the medical device. In other words, the patient carries a record on his treatment or survey constantly on him, and this record may in cases of emergency readily be accessed.

Advantageously, the medical treatment or monitoring process may be organized in a more flexible way, as the patient can update his therapy and manage his medical device at a personal computer without additional devices or adaptors and without any installation of specialized software. Accordingly the patient gains freedom to manage his medical device, e.g. whilst travelling.

The portable medical device, typically a drug delivery device, is worn by a user next to the skin or carried otherwise. In order to connect the medical device to a personal computer, the medical device is either dismounted from the user and then connected to the personal computer, or the connection is realized whilst the medical device is worn by the user. This may e.g. be achieved with a cable connection that is long enough, or by a wireless connection between the medical device and the computer, such as IR or Bluetooth. Advantageously, the connection consists of a connection which ensures a secure and fast connection between the personal computer and the medical device, and preferably which further allows for the transfer of electrical energy from the personal computer and the medical device, e.g. a USB connection or a FireWire connection. Especially advantageous is the use of a USB connection, as USB interfaces are found as standard interface in personal computers, so that virtually every personal computer can be used as a user interface for managing the medical device.

According to one embodiment of the present invention, the portable medical device may consist of two separable units: A reusable unit and a disposable unit. The disposable unit contains parts of the medical device that are depleted when the medical device is in use, such as e.g. the drug reservoir in a drug delivery device. The reusable unit contains parts of the medical device that can be reused for several times, such as e.g. a battery, a memory, a processor, or an interface of the medical device.

The advantage of providing a portable medical device with a disposable unit and a reusable unit is that valuable parts of the medical device can be used several times, which makes the use of the medical device more economic. For instance, in the case of drug delivery devices, only the parts contacting the drug (reservoir, parts of the pump, infusion member) have to be replaced regularly, and the other, more costly parts, can thus be reused.

When a new disposable unit is attached to the reusable unit, an electronic identification number of the disposable unit is verified by using a program and an electronic list provided in the reusable unit. According to an embodiment of the present invention, every time the portable medical device is connected to a personal computer that is connected to a global communications network such as the internet, the electronic list of the medical device is updated. Accordingly faulty disposable units can safely and quickly be identified. Advantageously, the patient may receive a warning signal, e.g. an audible alarm or visual warning displayed on the personal computer or on the medical device, in the case that a faulty disposable unit has been attached to his reusable unit. The provider of disposable units thus may actively recall faulty products that have been sold already, accordingly improving the safety of the patient. Reversely, if patients report problems with their medical devices, a central database may gather data on where, when, and which disposable units have been used, as such data may be transferred from the medical device to the personal computer via a global communications network to a server when the medical device is connected to the personal computer. Accordingly the provider of disposable units may collect inform information which allows them to provide safety information to users, e.g. to provide a warning message to users that are about to use a disposable unit from the same production batch.

The program provided in the portable medical device advantageously is configured such that it enables the sending of data from the personal computer to a printer or using a global communications network, such as the internet, to for example a server or as an email for example to a medical care centre. The sending of data obtained by the portable medical device, for example the insulin doses delivered over time to a patient with diabetes, can accordingly be supervised by a medical care centre. This can be used to send help to the patient when the medical device reports a value that is critical for the wellbeing of the patient.

Data sent by the portable medical device via the internet to a server may be stored in order to create a database. Preferably this database may be accessible by a doctor or medical centre. It serves as a library of data related to the medical device, to which the patient and the doctor both have access. When the portable medical device storage capacity is reached, said database may store older data and allow deleting older data on the medical device in order to create new storage capacity. If the older data is required again, it can be downloaded from the server to the medical device.

Furthermore, data obtained by the portable medical device which is regularly sent to an evaluation unit can be used for medical studies in an easy and convenient way.

According to another aspect of the present invention, the connection of the portable medical device through a personal computer to the internet and to other servers can be used to download information from a server to the medical device. This feature allows a doctor to adjust the settings of a medical device, so that the patient receives a customized and updated therapy.

The program in the portable medical device may further be configured to search for software updates on the internet when the medical device is connected to a personal computer which is connected to the internet. This feature allows the patient to use new software releases without having to search for it. Moreover, it allows fixing faulty programs, such as the steering software or firmware.

According to one embodiment, the program in the portable medical device may be further configured to process data obtained by another medical device. E.g. when an insulin delivery device is managed on a personal computer, the patient at the same time is usually monitoring his blood glucose level. The program in the portable medical device now is configured to include data from a foreign medical device into the graphs and figures, in order to gain a more complete picture of the condition of the patient or the status of the treatment.

In a preferred embodiment, the battery contained in the portable medical device may be recharged when the medical device is connected to a personal computer, whereby, the program in the medical device is configured to enable charging of the medical device battery when a current supporting connection is established between the medical device and a personal computer. The connection between the medical device and the personal computer may be any connection which allows for the transfer of electrical energy from the personal computer to the medical device, for instance, a USB connection or a FireWire connection. In the present invention, smaller and lighter batteries can be used for the portable medical device, while enjoying high autonomy, since the batteries can be automatically and regularly recharged each time the device is connected to a personal computer. The portable medical device can thus be as light and as small as possible to be easy to carry and ensure convenient use

There is also provided a method of analyzing at least one parameter of a medical device, the method comprising the steps of a) providing a portable medical device comprising a memory, a processor, a medical device interface, and a program configured to process data obtained or generated by the portable medical device into a format readable by a personal computer browser or spreadsheet program, b) connecting the portable medical device interface to a personal computer interface, and c) displaying at least one parameter of the portable medical device on the personal computer.

Further objects and advantageous aspects of the invention will be apparent from the claims and the following description of embodiments of the invention in conjunction with the drawings in which:
Fig.1 shows a schematic view of a portable medical device connected to a personal computer for the management of the medical device according to an embodiment of the invention,
Fig.2 shows a schematic view of a portable medical device according to an embodiment of the present invention connected to a personal computer and to a remote server via a global communications network
Fig.3 shows a schematic view of a portable medical device connected to a personal computer for recharging the batteries of the medical device according to an embodiment of the invention,
Fig.4 shows a schematic view of the process control in a portable medical device according to an embodiment of the invention, and
Fig.5 shows a schematic view of a portable medical device, according to an embodiment of the invention.

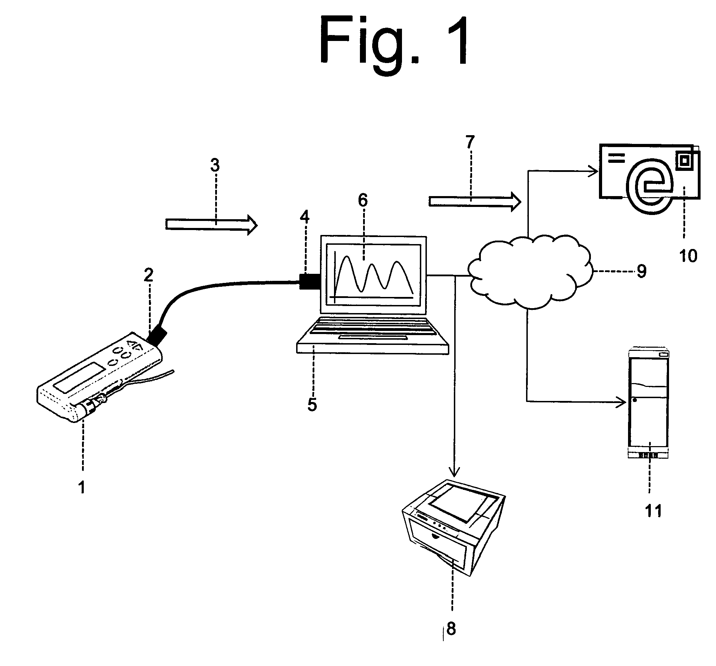

Referring to figure 1, a portable medical device 1 according to the present invention is connected to a personal computer 5. The medical device interface 2 is connected by means of a cable to the personal computer interface 4. The connection is advantageously a USB or FireWire connection. After establishing the connection, the personal computer 5 recognizes that the portable medical device 1 has been connected. This triggers the opening of a window on the monitor 6 of the personal computer 5, and a program stored on the portable medical device 1 is run. In other words, the personal computer 5 serves as a user interface (monitor 6, keyboard, and mouse or track pad) which enables the user to manage his medical device 1.

As no specific programs need to be installed on the personal computer 5, the user does not need to possess administrator rights for the personal computer 5. This is of particular interest when using foreign personal computers, such as computers in internet cafes.

Preferably, various types of diagrams are stored on the portable medical device 1, so that the user has the option to choose the type of diagram which serves him best. As the various types of diagrams are stored on the portable medical device 1, there is no need for specialized software on the personal computer 5 to transform one type of diagram into another type of diagram.

As shown in Fig.1 the personal computer 5 further may advantageously be connected to a data output device 8, or to a global communications network 9, such as the internet. In such a system, the information stored on the portable medical device 1 can not only be shown on the monitor 6 of a personal computer 5, but also be printed out by a data output device 8 or transferred through a global communications network 9 to a server 11 or sent as an email 10. This data transfer 7 from the personal computer to other devices allows e.g. the gathering of medial data for a clinical study, or the information of a doctor or medical centre on the actual condition of his patient.

As internet connection and data output devices are readily available, these functions of the present invention do not depend on specialized software or additional devices.

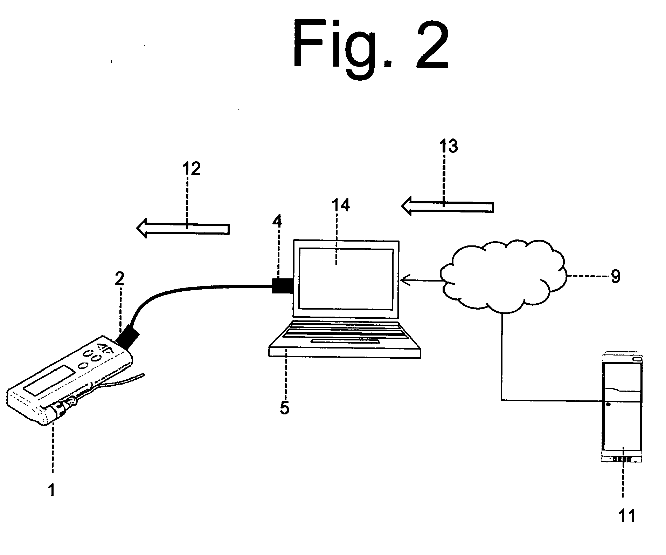

Referring to figure 2, a medical device 1 according to the present invention is shown connected to a personal computer 5 which has a connection with a server 11 through a global communications network 9, e.g. the internet. According to one preferred embodiment of the invention the portable medical device 1 contains a program that is configured such that the personal computer 5 is triggered to receive data ,such as for example, medical device parameters, updated treatment schedule information, product information etc, from the server 11 through the global communications network 9 on connection of the medical device to the personal computer. The data 12 then is transferred from the personal computer 5 to the medical device 1. Preferably the medical device program is configured to provide an input mask displayed on the monitor 6 of the personal computer, which allows the user to select which part(s) of the data supplied to the computer shall be transferred to the medical device 1.

Accordingly, parameters of the medical device 1, such as e.g. the ratio of drug delivery, can be updated easily and conveniently by transferring data from a server 11 to the medical device1. This allows adjusting medical treatments over time, as the medical device 1 is connected to the personal computer 5 e.g. every few days.

According to an embodiment of the present invention, the portable medical device 1 may advantageously contain a program that is configured such that, when the portable medical device is connected to the personal computer 5, the computer searches for software updates and receives software updates from the server 11 through the global communications network 9. Preferably the portable medical device program is configured to provide an input mask displayed on the monitor 6 of the personal computer, which allows the user to select whether to software update retrieved by the personal computer shall be copied to the medical device 1 for installation or not. The selected software update then is installed on the portable medical device 1.

Accordingly, patients using such a medical device have the opportunity to use new software releases as soon as they become available on the internet.

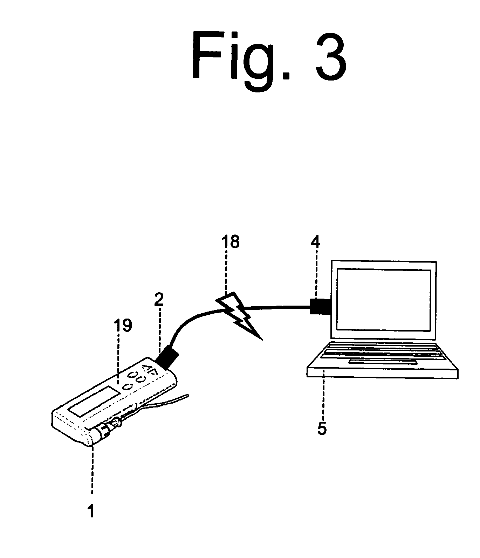

Referring to figure 3, a portable medical device 1 is connected to a personal computer 5 for recharging the battery of the portable medical device 1 according to an embodiment of the invention. In the embodiment illustrated, a program in the portable medical device 1 is configured such that it initiates a power transmission 18 from the personal computer 5 to the medical device 1. A rechargeable battery 19 is charged utilizing the power transmission 18. A program contained in the medical device 1 controls the recharging of the battery 19, so that the power transmission is adjusted to the actual charging level of the battery 19.

Accordingly, the battery 19 in the portable medical device 1 may be smaller and lighter than batteries for the same purpose that are not recharged, because every time the medical device 1 is connected to the personal computer 5, the battery 19 is recharged. Both small size and lightness contribute to the wearing comfort of the medical device 1.

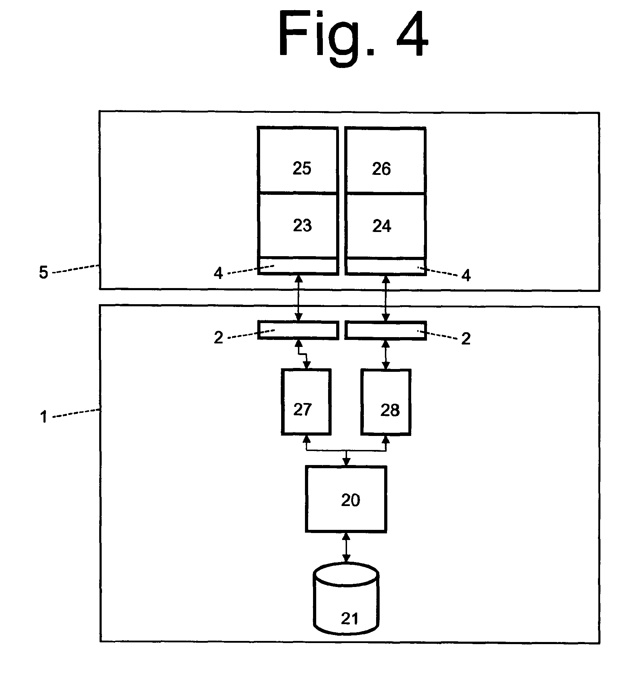

Referring to figure 4, the process control in a portable medical device 1 according to an embodiment of the invention is illustrated schematically. The memory 21 is accessed by the processor 20, which processes data obtained or generated by the portable medical device 1 into a format readable by a personal computer browser or spreadsheet program. This processed data 27, 28, which preferably consists of data in an xml or html type format, then is transferred from the medical device interface 2 to the personal computer interface 4. Over the data access 23, 24 in the personal computer 5, the data is then displayed using a standard personal computer application 25, 26. In a preferred embodiment, an internet browser or a spreadsheet program is used to display the data on the monitor 6 of the personal computer 5.

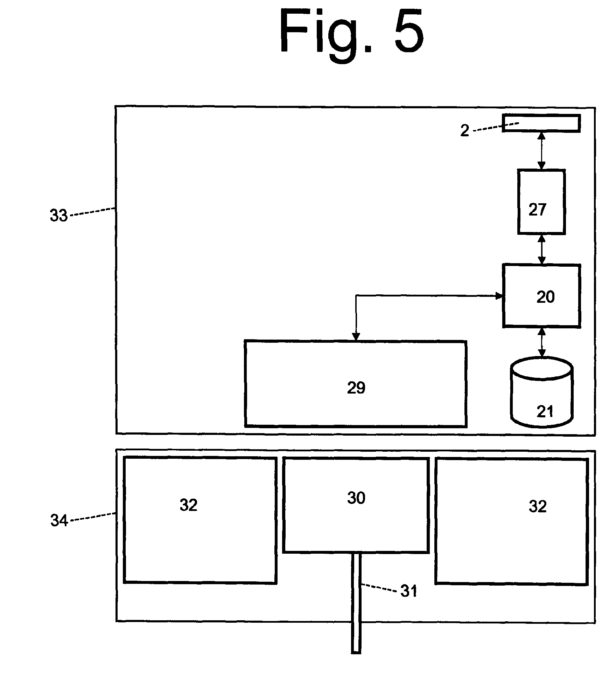

Referring to figure 5, according to one embodiment of the invention the portable medical device consists of a reusable unit 33 and a disposable unit 34. In the embodiment illustrated, the medical device 1 is a drug delivery device. A pump 30, an infusion member 31, and a drug reservoir 32 are comprised in the disposable unit 34, whereas a pump drive 29, a memory 21, a processor 20, a medical device interface 2 are contained in the reusable unit 33. The disposable unit 34 and the reusable unit 33 can be disengaged, so that the disposable unit 34 may be disposed off, and a new disposable unit 34 may be attached to the reusable unit 33.

In the case of medical devices used for drug administration, all parts coming into contact with the drug and with the patient typically have to be disposed off after a certain period of time. With a preferred design as disclosed in this embodiment of the invention, only cheap parts, such as a reservoir 32, a pump 30, and an infusion member 31, have to be disposed off regularly, whereas more expensive parts, such as a pump drive 29, a memory 21, a processor 20 and a medical device interface 2, can be used over a longer period of time.

Alternatively, according to another embodiment of this invention, the reusable unit 33 may be a hand held device which controls the disposable unit 34. The hand held device and the disposable unit 34, e.g. a patch attached to the users skin, together constitute the portable medical device 1. Preferably, the hand held device and the patch communicate wirelessly together. Alternatively a cable connection may connect the hand held device to the patch in order to allow communication.

## Claims

1. A portable medical device (1) comprising a memory (21), a processor (20), a medical device plug and play interface (2), and a program configured to process data obtained or generated by the medical device (1) into a format directly displayable by a personal computer without installation of any software or hardware dedicated to the medical device.

2. A portable medical device (1) according to claim 1, **characterized in that** the data transferred from the medical device to the personal computer contains data in xml or html format.

3. A portable medical device (1) according to the previous claim, **characterized in that** the program is configured to automatically initiate a computer browser or spreadsheet program of a personal computer (5) to display the processed data when the medical device interface (2) is connected to a personal computer interface (4).

4. A portable medical device (1) according to anyone of the previous claims, **characterized in that** a connection between the medical device interface (2) and a computer interface (4) is a USB connection.

5. A portable medical device (1) according to anyone of the previous claims, **characterized in that** a battery in the medical device (1) is configured to be recharged when the medical device interface (2) is connected to a computer interface (4).

6. A portable medical device (1) according to anyone of the previous claims, **characterized in that** the medical device is an insulin delivery device.

7. A portable medical device (1) according to the previous claim, **characterized in that** the medical device comprises a program configured to download a value of at least one parameter of the medical device from a server (11) via a global communications network (9) when the medical device (1) is connected to a personal computer (5).

8. A portable medical device (1) according to anyone of the previous claims, **characterized in that** the medical device comprises a disposable unit (34) comprising a drug reservoir (32), and a reusable unit (33) comprising a memory (21), a processor (20), a medical device interface (2), and a program configured to process data obtained or generated by the medical device (1) into a format directly displayable by a personal computer without installation of any medical device specific software or hardware.

9. A method of managing a portable medical device (1), the method comprising:
providing a medical device (1) comprising a memory (21), a processor (20), a medical device plug and play interface (2), and a program configured to process data obtained or generated by the medical device (1) into a format directly displayable by a personal computer without installation of any medical device specific software or hardware;
connecting the medical device interface (2) to a personal computer interface (4); displaying at least one parameter of the medical device (1) on a monitor (6) of the personal computer (5).

10. A method for adjusting at least one parameter of a portable medical device (1), the method comprising:
providing a portable medical device according to claim 1;
providing in said portable medical device a program configured to download values for at least one parameter of the medical device from a server (11) connected via a global communications network to the personal computer (5)
connecting a medical device interface (2) to a personal computer interface (4); downloading values for at least one parameter of the medical device from said server (11) into said portable medical device via said personal computer connected to said global communications network.

11. A method for installing a medical device program in a portable medical device (1), the method comprising:
providing a portable medical device according to claim 1;
providing in said portable medical device a program configured to search for said medical device program stored on a server (11);
connecting the medical device interface (2) to a personal computer interface (4); connecting the personal computer to the server (11) via a global communications network;
searching for and downloading the medical device program found on the server (11);
installing the medical device program on the portable medical device (1).

12. A method for warning a patient when at least one parameter of a medical device (1) is outside of a predefined range, the method comprising:
providing a portable medical device (1) comprising a memory (21), a processor (20), a medical device plug and play interface (2), a program configured to process data obtained or generated by the medical device (1) into a format directly displayable by a personal computer without installation of any software or hardware dedicated to the medical device, and a program that is configured to activate an alarm when at least one parameter obtained or generated by the medical device (1) is outside the predefined range ;
predefining at least one range in which at least one parameter must be located if a condition of a patient is healthy or a therapy on schedule.
